# FASCICULE DE BREVET EUROPEEN

(11) **EP 1 127 047 B1**
(45) Date de publication et mention de la délivrance du brevet: **10.09.2003**
(21) Numéro de dépôt: 99954042.0
(22) Date de dépôt: 03.11.1999
(51) Int. Cl.: C07C 255/24, C07C 253/30

(54) **PROCEDE D'HEMIHYDROGENATION DE DINITRILES**
VERFAHREN ZUR HEMIHYDRIERUNG VON DINITRILEN
HEMIHYDROGENATION METHOD FOR DINITRILES

(30) Priorité: 05.11.1998 FR 9814100
(43) Date de publication de la demande: 29.08.2001
(73) Titulaire: Rhodia Polyamide Intermediates, 69192 Saint-Fons (FR)
(72) Inventeur: BOSCHAT, Vincent, F-69007 Lyon (FR); BRUNELLE, Jean-Pierre, F-78290 Croissy sur Seine (FR); BOBET, Jean-Louis, F-33600 PESSAC (FR); DARRIET, Bernard, F-33650 SAUCATS (FR); CHEVALIER, Bernard, F-33400 TALENCE (FR)
(74) Mandataire: Esson, Jean-Pierre
(86) Numéro de dépôt international: FR9902677
(87) Numéro de publication internationale: WO00027806

(56) Documents cités:
- WO-A-93/12073
- WO-A-93/16034
- WO-A-96/18603
- WO-A-96/20166

## Description

La présente invention concerne l'hydrogénation d'une fonction nitrile d'un dinitrile, afin d'obtenir l'aminonitrile correspondant.

Généralement l'hydrogénation des dinitriles est réalisée pour préparer les diamines correspondantes ; ainsi particulièrement l'hydrogénation de l'adiponitrile conduit à l'hexaméthylène diamine, qui est l'un des deux composés de base de la préparation du polyamide-6,6.

Cependant, il s'avère parfois nécessaire de préparer non la diamine, mais l'aminonitrile intermédiaire. C'est par exemple, mais non limitativement, le cas pour l'hémihydrogénation de l'adiponitrile en aminocapronitrile, composé susceptible d'être ensuite transformé en caprolactame, produit de base du polyamide-6, ou directement en polyamide-6.

Ainsi le brevet US 4 389 348 décrit un procédé d'hydrogénation de dinitrile en oméga-aminonitrile, par l'hydrogène, en milieu solvant aprotique et ammoniac et en présence de rhodium déposé sur un support basique.

Le brevet US 5 151 543 décrit un procédé d'hydrogénation partielle de dinitriles en aminonitriles dans un solvant en excès molaire d'au moins 2/1 par rapport au dinitrile, comprenant de l'ammoniac liquide ou un alcanol contenant une base minérale soluble dans ledit alcanol, en présence d'un catalyseur de type nickel ou cobalt de Raney.

La demande de brevet WO-A-96/18603 décrit un procédé d'hémihydrogénation de dinitriles aliphatiques en aminonitriles correspondants, à l'aide d'hydrogène et en présence d'un catalyseur choisi parmi le nickel de Raney ou le cobalt de Raney, ledit nickel ou cobalt de Raney comportant de préférence un élément dopant tel que le chrome, le fer, le titane ou le zinc, et d'une base minérale forte dérivant d'un métal alcalin ou alcalino-terreux, le milieu initial d'hydrogénation comportant de l'eau à raison d'au moins 0,5 % en poids par rapport à la totalité des composés liquides dudit milieu, de la diamine et/ou de l'aminonitrile susceptibles de se former à partir du dinitrile à hydrogéner ainsi que du dinitrile non transformé à raison pour l'ensemble de ces trois composés de 80 % à 99,5 % en poids par rapport à la totalité des composés liquides dudit milieu.

Comme cela ressort de l'analyse qui précède, les procédés d'hémihydrogénation de dinitriles en aminonitriles mettent le plus souvent en oeuvre un catalyseur de type Raney, nickel ou cobalt, de préférence dopé par la présence d'un ou de plusieurs autres éléments (voir aussi WO-A-96/20166).

Il a maintenant été trouvé que la présence de cuivre et/ou d'argent et/ou d'or dans le nickel ou le cobalt de Raney conduit de manière inattendue à d'excellents résultats en termes de sélectivité en aminonitrile.

L'hydrogénation sélective d'une seule fonction nitrile d'un dinitrile est par nature très difficile à réaliser, car l'aminonitrile est le composé intermédiaire de l'hydrogénation complète en diamine. Cet intermédiaire entre donc en compétition avec le dinitrile de départ comme composé susceptible d'être hydrogéné. Par conséquent la répartition globale entre aminonitrile et diamine obtenus résulte du rapport des coefficients cinétiques d'hydrogénation de la première fonction nitrile (k1) et de la deuxième fonction nitrile (k2).

Il a été constaté que l'utilisation d'un cobalt ou d'un nickel de Raney dopé par au moins un métal choisi parmi le cuivre, l'argent et l'or permet d'améliorer le rapport k1/k2 de ces coefficients cinétiques et donc la sélectivité de l'hydrogénation en aminonitrile par rapport à l'hydrogénation complète en diamine, en comparaison avec la mise en oeuvre d'un catalyseur de Raney dopé avec les éléments les plus fréquemment utilisés comme par exemple le chrome, le fer ou le titane.

La présente invention a trait à l'hydrogénation préférentielle d'une seule fonction nitrile d'un dinitrile (aussi appelée dans le présent texte hémihydrogénation) de manière à préparer majoritairement l'aminonitrile correspondant et seulement de manière minoritaire la diamine.

Plus précisément, elle concerne un procédé d'hémihydrogénation d'un dinitrile en aminonitrile correspondant, dans un milieu liquide, caractérisé en ce que l'on opère en présence d'un catalyseur nickel ou cobalt de Raney contenant au moins un métal choisi parmi le cuivre, l'argent et l'or et en présence d'un hydroxyde de métal alcalin ou alcalino-terreux.

Les nickels de RANEY sont des catalyseurs largement utilisés dans l'industrie pour les réactions d'hydrogénation. Ils sont préparés par attaque alcaline d'alliages Al/Ni ou Al/Co riches en aluminium et contenant le cas échéant d'autres métaux, généralement appelés dopants ou promoteurs. Le catalyseur est constitué par des agglomérats de cristallites de nickel ou de cobalt à grande surface spécifique et à concentration en aluminium résiduel variable.

Ce catalyseur comprend généralement une teneur en aluminium, exprimée en poids par rapport au poids du nickel ou du cobalt, inférieure ou égale à 10 %.

Avantageusement, le rapport pondéral (Cu + Ag + Au)/Ni ou (Cu + Ag + Au)/Co du catalyseur utilisé dans la présente invention est compris entre 0,05 % et 10 % et de préférence entre 0,1 % et 5 % .

Avec le cuivre et/ou l'argent et/ou l'or, le catalyseur mis en oeuvre dans le procédé peut contenir des quantités, généralement plus faibles, d'un ou de plusieurs autres éléments également rassemblés sous le terme générique de dopant ou de promoteur.

Ces dopants complémentaires éventuellement présents, sont choisis de préférence parmi les éléments suivants : titane, chrome, fer, zirconium, vanadium, manganèse, bismuth, tantale, rhodium, ruthénium, iridium, platine, palladium, niobium, hafnium, les éléments des terres rares. Lorsque le catalyseur mis en oeuvre est le nickel de Raney, le cobalt peut également être présent comme dopant complémentaire. De même, lorsque le catalyseur mis en oeuvre est le cobal de Raney, le nickel peut également être présent comme dopant complémentaire. Le titane, le chrome, le fer et le zirconium sont particulièrement préférés.

La quantité de dopant autre que le cuivre, l'argent et l'or que peut contenir le catalyseur représente généralement de 0 % à 5 % en poids du poids du nickel ou du cobalt.

Parmi les catalyseurs de type Raney utilisés, on préfèrera le nickel de Raney tel que défini précédemment.

Le catalyseur utilisé dans la présente invention peut également être mis en oeuvre sous forme de grains.

Le procédé de l'invention s'applique, plus particulièrement mais non limitativement, aux substrats dinitriles de formule (I) :

NC―R―CN (I)

dans laquelle R représente un groupement alkylène, linéaire ou ramifié, ayant de 1 à 12 atomes de carbone.

De préférence, on met en oeuvre dans le procédé de l'invention des dinitriles de formule (I) dans laquelle R représente un radical alkylène, linéaire ou ramifié, ayant de de 1 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile ainsi que des mélanges de plusieurs de ces dinitriles, notamment les mélanges adiponitrile, méthylglutaronitrile, éthylsuccinonitrile qui proviennent d'un même procédé de synthèse de l'adiponitrile.

La concentration du milieu réactionnel en dinitriles, notamment en adiponitrile peut varier largement selon le mode de mise en oeuvre de l'invention, marche continue ou discontinue, charge initiale ou introduction progressive par exemple. Dans le cadre préféré d'un procédé industriel en marche continue, la concentration moyenne en dinitriles se situe habituellement entre 10 % et 45 % en poids par poids.

Les hydroxydes de métal alcalin, LiOH, NaOH, KOH, RbOH, CsOH et leurs mélanges, sont utilisés de préférence.

En pratique, on utilise préférentiellement NaOH et KOH, pour un bon compromis performance-prix.

Le milieu réactionnel d'hydrogénation est liquide. Il contient au moins un solvant apte à solubiliser au moins partiellement le substrat dinitrile à hydrogéner.

Suivant une modalité intéressante du procédé selon l'invention, on utilise un milieu réactionnel liquide au moins partiellement aqueux. L'eau représente généralement une quantité de 1 % à 25 % en poids par rapport au poids du milieu réactionnel total. De préférence, la teneur en eau du milieu réactionnel est comprise entre 1 et 15 % en poids par poids.

En complément ou en substitution à l'eau, on peut prévoir au moins un autre solvant, généralement de type alcool. Les alcools qui conviennent plus particulièrement sont par exemple le méthanol, l'éthanol, le propanol, l'isopropanol, le butanol et les mélanges desdits composés.

Lorsqu'il est employé avec l'eau, le solvant alcoolique représente de deux à quatre parties en poids pour une partie en poids d'eau et de préférence trois parties pour une partie d'eau.

Selon une autre caractéristique préférée de l'invention, le milieu initial d'hydrogénation comprend, en particulier dans le cadre d'une mise en oeuvre continue du procédé, de la diamine qui est coproduite par l'hydrogénation. Il s'agit par exemple d'hexaméthylènediamine lorsque le substrat dinitrile est l'adiponitrile.

La concentration moyenne en régime continu de l'aminonitrile et/ou de la diamine dans le milieu réactionnel est avantageusement comprise entre 35 % et 90 % en poids par rapport au poids de la totalité du solvant inclus dans ledit milieu réactionnel et, plus préférentiellement, est comprise entre 45 % et 89 % en poids par poids.

Le milieu réactionnel peut comporter de l'ammoniac liquide ou dissous. Généralement l'ammoniac représente de 0 % à 50 % en poids du milieu réactionnel et de préférence de 0 % à 15 %.

La quantité d'hydroxyde alcalin ou alcalino-terreux dans le milieu réactionnel varie en fonction de la nature dudit milieu réactionnel.

Dès lors que le milieu réactionnel ne contient que de l'eau, les produits de la réaction et éventuellement de l'ammoniac, à titre de milieu solvant liquide, la quantité d'hydroxyde de métal alcalin ou alcalino-terreux est avantageusement supérieure ou égale à 0,1 mol/kg de catalyseur, de préférence comprise entre 0,1 et 2 mol/kg de catalyseur et plus préférentiellement encore entre 0,2 et 1,0 mol/kg de catalyseur.

Dans le cas où le milieu réactionnel comprend également un alcool, la quantité d'hydroxyde de métal alcalin ou alcalino-terreux est supérieure ou égale à 0,05 mol/kg de catalyseur, est comprise de préférence entre 0,1 et 10,0 mol/kg et plus préférentiellement encore entre 1,0 et 8,0 mol/kg.

La température à laquelle est réalisé le procédé d'hémihydrogénation est généralement inférieure ou égale à 150°C, de préférence inférieure ou égale à 120°C et, plus préférentiellement encore, inférieure ou égale à 100°C.

Concrètement, cette température est le plus souvent comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 0,10 et 10 MPa.

La durée de la réaction est variable en fonction des conditions réactionnelles et du catalyseur.

Dans un mode de fonctionnement discontinu, elle peut varier de quelques minutes à plusieurs heures.

Dans un mode de fonctionnement continu, qui est préféré pour le procédé selon l'invention, la durée n'est évidemment pas un paramètre figeable.

Il est à noter que l'homme du métier peut moduler la chronologie des étapes du procédé selon l'invention, selon les conditions opératoires. L'ordre donné ci-avant ne correspond qu'à une forme préférée, mais non limitative, du procédé selon l'invention.

Les autres conditions qui régissent l'hydrogénation (en mode continu ou discontinu) conforme à l'invention, relèvent de dispositions techniques traditionnelles et connues en elles-mêmes.

Grâce à toutes les dispositions avantageuses évoquées ci-dessus, le procédé de l'invention permet d'hydrogéner des substrats dinitriles en aminonitriles correspondants, de façon sélective, rapide, commode et économique.

L'hémihydrogénation de l'adiponitrile fournit l'amino-6 capronitrile, composé qui est aisément transformable par hydrolyse cyclisante en caprolactame, produit de départ de la synthèse du polyamide-6.

L'invention est illustrée par les exemples qui suivent d'hémihydrogénation d'adiponitrile en amino-6 capronitrile.

Dans ces exemples les abréviations suivantes pourront être utilisées :
- ADN = adiponitrile
- ACN = aminocapronitrile
- HMD = hexaméthylène diamine
- ACA = aminocaproamide
- CVA = cyanovaléramide
- TT = taux de transformation
- RT = sélectivité par rapport au substrat de départ transformé (ici par rapport à l'ADN)
- RR = rendement par rapport au substrat (ADN) de départ engagé
- IPOL = indice polarographique traduisant la présence d'impuretés notamment de type imine qui provoquerent en particulier des colorations et des ramifications lors de la polymérisation du caprolactame (préparé par hydrolyse de l'ACN), si elles se retrouvent dans ce dernier ; cet indice polarographique est donc déterminé par polarographie et est exprimé en moles de fonction imine par tonne d'échantillon à doser.

### EXEMPLE 1

Dans un réacteur en acier inoxydable de 100 ml, équipé d'une agitation de type rushtone cavitator, de moyens d'introduction des réactifs et de l'hydrogène et d'un système de régulation de température, on charge :
- adiponitrile 24,0 g
- hexaméthylène diamine 24,0 g
- eau 5,3 g
- KOH 0,064 g
- Ni de Raney (à 1,7 % de Cu) 1,35 g

On chauffe le mélange réactionnel à 50°C après avoir purgé le réacteur à l'azote, puis à l'hydrogène ; la pression est alors réglée à 2,5 MPa à cette température par addition continue d'hydrogène. L'avancement de la réaction est suivi par la consommation d'hydrogène et l'analyse par chromatographie en phase vapeur (CPG) d'un prélèvement du mélange réactionnel. Lorsque l'optimum de rendement est atteint, on arrête la réaction par arrêt de l'agitation et refroidisssement du mélange réactionnel.

On obtient les résultats suivants :
- durée de la réaction : 321 min
- TT de l'ADN : 82,3 %
- RR en ACN : 60,3 %
- RR en HMD : 20,9 %
- Somme des RT en ACN et HMD : 98,7 %
- indice IPOL : 15,0

Le complément à 100 % des RT et des composés évalués par l'indice IPOL est représenté par de l'ACA et du CVA qui sont des composés valorisables lors de l'hydrolyse cyclisante de l'ACN en caprolactame.

### EXEMPLE 2

On répète l'exemple 1 dans le même appareillage, avec les mêmes quantités de réactifs et dans les mêmes conditions, mais en utilisant du Ni de Raney comportant 3 % en poids de Cu.

On obtient les résultats suivants :
- durée de la réaction : 395 min
- TT de l'ADN : 85,7 %
- RR en ACN : 58,9 %
- RR en HMD : 21,0 %
- Somme des RT en ACN et HMD : 93,2 %
- indice IPOL : 19,6

Le complément à 100 % des RT et des composés évalués par l'indice IPOL est représenté par de l'ACA et du CVA.

### EXEMPLE 3

On répète l'exemple 1 dans le même appareillage, avec les mêmes quantités de réactifs et dans les mêmes conditions mais en utilisant du Ni de Raney comportant 3% en poids de cuivre et 2,1% en poids de chrome.

On obtient les résultats suivants :

| | |
|---|---|
| Durée de la réaction | 29 min |
| TT de l'ADN | 80,3% |
| RR de l'ACN | 56,3% |
| RR de l'HMD | 24,5% |
| Somme des RT en ACN et HMD | 100% |
| Indice IPOL | 26 |

### ESSAI COMPARATIF 1

On répète l'exemple 1 dans le même appareillage, avec les mêmes quantités de réactifs et dans les mêmes conditions, mais en utilisant du Ni de Raney comportant 1,7 % en poids de Chrome.

On obtient les résultats suivants :
- durée de la réaction : 30 min
- TT de l'ADN : 81,7 %
- RR en ACN : 56,9 %
- RR en HMD : 22,4 %
- Somme des RT en ACN et HMD : 97,1 %
- indice IPOL : 72,5

Le complément à 100 % des RT et des composés évalués par l'indice IPOL est représenté par des sous-produits non pris en compte dans l'indice IPOL et autres que l'ACA et du CVA (par exemple le bis-hexaméthylènetriamine).

## Revendications

1. Procédé d'hémihydrogénation d'un dinitrile en aminonitrile correspondant; dans un milieu liquide, **caractérisé en ce que** l'on opère en présence d'un catalyseur nickel ou cobalt de Raney contenant au moins un métal choisi parmi le cuivre, l'argent et l'or et en présence d'un hydroxyde de métal alcalin ou alcalino-terreux.

2. Procédé selon la revendication 1, **caractérisé en ce que** le rapport pondéral (Cu + Ag + Au)/Ni ou (Cu + Ag + Au)/Co du catalyseur utilisé est compris entre 0,05 % et 10 % et de préférence entre 0,1 % et 5 %.

3. Procédé selon l'une des revendications 1 ou 2, **caractérisé en ce que** le catalyseur contient un ou plusieurs dopants complémentaires choisis parmi le titane, le chrome, le fer, le zirconium, le vanadium, le manganèse, le bismuth, le tantale, le rhodium, le ruthénium, l'iridium, le platine, le palladium, le niobium, l'hafnium, les éléments des terres rares, ainsi que le cobalt lorsque le catalyseur est le nickel de Raney et le nickel lorsque le catalyseur est le cobal de Raney.

4. Procédé selon l'une des revendications 1 à 3, **caractérisé en ce que** la quantité de dopant autre que le cuivre, l'argent et l'or que contient le catalyseur représente de 0 % à 5 % en poids du poids du nickel ou du cobalt.

5. Procédé selon l'une des revendications 1 à 4, **caractérisé en ce que** le catalyseur utilisé est de préférence un nickel de Raney.

6. Procédé selon l'une des revendications 1 à 5, **caractérisé en ce que** le catalyseur est mis en oeuvre sous forme de grains.

7. Procédé selon l'une des revendications 1 à 6, **caractérisé en ce qu'**il s'applique aux substrats dinitriles de formule (I) :
NC―R―CN (I)
dans laquelle R représente un groupement alkylène, linéaire ou ramifié, ayant de 1 à 12 atomes de carbone et de préférence un radical alkylène, linéaire ou ramifié, ayant de de 1 à 6 atomes de carbone.

8. Procédé selon l'une des revendications 1 à 7, **caractérisé en ce que** la concentration moyenne du milieu réactionnel en dinitrile dans le cadre d'un procédé industriel en marche continue se situe entre 10 % et 45 % en poids par poids.

9. Procédé selon l'une des revendications 1 à 8, **caractérisé en ce qu'**il est mis en oeuvre en présence de LiOH, NaOH, KOH, RbOH, CsOH et de leurs mélanges.

10. Procédé selon l'une des revendications 1 à 9, **caractérisé en ce que** le milieu réactionnel liquide est au moins partiellement aqueux et l'eau représente une quantité de 1 % à 25 % en poids par rapport au poids du milieu réactionnel total et de préférence est comprise entre 1 et 15 % en poids par poids.

11. Procédé selon l'une des revendications 1 à 10, **caractérisé en ce que** le milieu réactionnel comporte, en complément ou en substitution à l'eau, au moins un autre solvant de type alcool.

12. Procédé selon la revendication 11, **caractérisé en ce que** milieu réactionnel comporte un alcool choisi parmi le méthanol, l'éthanol, le propanol, l'isopropanol, le butanol et les mélanges desdits composés.

13. Procédé selon l'une des revendications 1 à 12, **caractérisé en ce que** le milieu réactionnel comporte de la diamine qui est coproduite par l'hydrogénation.

14. Procédé selon la revendication 13, **caractérisé en ce que** la concentration moyenne en régime continu de l'aminonitrile et/ou de la diamine dans le milieu réactionnel est comprise entre 35 % et 90 % en poids par rapport au poids de la totalité du solvant inclus dans ledit milieu réactionnel et, plus préférentiellement, est comprise entre 45 % et 89 % en poids par poids.

15. Procédé selon l'une des revendications 1 à 14, **caractérisé en ce que** le milieu réactionnel comporte l'ammoniac liquide ou dissous.

16. Procédé selon l'une des revendications 1 à 15, **caractérisé en ce que** l'ammoniac représente de 0 % à 50.% en poids du milieu réactionnel et de préférence de 0 % à 15 %.

17. Procédé selon l'une des revendications 1 à 10 et 13 à 16, **caractérisé en ce que** la quantité d'hydroxyde de métal alcalin ou alcalino-terreux est supérieure ou égale à 0,1 mol/kg de catalyseur, de préférence comprise entre 0,1 et 2 mol/kg de catalyseur et plus préférentiellement encore entre 0,2 et 1,0 mol/kg de catalyseur.

18. Procédé selon l'une des revendications 11 et 12, **caractérisé en ce que** la quantité d'hydroxyde de métal alcalin ou alcalino-terreux est supérieure ou égale à 0,05 mol/kg de catalyseur, de préférence comprise entre 0,1 et 10,0 mol/kg de catalyseur et plus préférentiellement entre 1,0 et 8,0 mol/kg de catalyseur.

19. Procédé selon l'une des revendications 1 à 18, **caractérisé en ce qu'**il est réalisé à une température inférieure ou égale à 150°C, de préférence inférieure ou égale à 120°C et, plus préférentiellement comprise entre la température ambiante (20°C environ) et 100°C.

20. Procédé selon l'une des revendications 1 à 19, **caractérisé en ce qu'**il est réalisé sous une pression en hydrogène comprise entre 0,10 et 10 MPa.

## Patentansprüche

1. Verfahren zur Hemihydrierung eines Dinitrils zu entsprechendem Aminonitril in einem flüssigen Medium, **dadurch gekennzeichnet, dass** man in Gegenwart eines Raney-Nickel- oder Raney-Kobaltkatalysators, der wenigstens ein Metall enthält, das ausgewählt ist unter Kupfer, Silber und Gold, und in Gegenwart eines Alkali- oder Erdalkalimetallhydroxids arbeitet.

2. Verfahren gemäß Anspruch 1, **dadurch gekennzeichnet, dass** das Gewichtsverhältnis (Cu + Ag + Au)/Ni oder (Cu + Ag + Au)/Co des verwendeten Katalysators zwischen 0,05 % und 10 % und vorzugsweise zwischen 0,1 % und 5 % liegt.

3. Verfahren gemäß einem der Ansprüche 1 oder 2, **dadurch gekennzeichnet, dass** der Katalysator ein oder mehrere zusätzliche Dotierungsmittel enthält, die ausgewählt sind unter Titan, Chrom, Eisen, Zirkonium, Vanadium, Mangan, Bismut, Tantal, Rhodium, Ruthenium, Iridium, Platin, Palladium, Niob, Hafnium, den Seltenerdelementen sowie Kobalt, wenn der Katalysator Raney-Nickel ist, und Nickel, wenn der Katalysator Raney-Kobalt ist.

4. Verfahren gemäß einem der Ansprüche 1 bis 3, **dadurch gekennzeichnet, dass** die Menge an von Kupfer, Silber und Gold verschiedenem Dotierungselement, die der Katalysator enthält, 0 Gew.-% bis 5 Gew.-% des Gewichts von Nickel oder Kobalt darstellt.

5. Verfahren gemäß einem der Ansprüche 1 bis 4, **dadurch gekennzeichnet, dass** der verwendete Katalysator vorzugsweise ein Raney-Nickel ist

6. Verfahren gemäß einem der Ansprüche 1 bis 5, **dadurch gekennzeichnet, dass** der Katalysator in Form von Körnern eingesetzt wird.

7. Verfahren gemäß einem der Ansprüche 1 bis 6, **dadurch gekennzeichnet, dass** es auf Dinitrilsubstrate der Formel (I):
NC-R-CN (I)
angewandt wird, worin R eine lineare oder verzweigte Alkylengruppe mit 1 bis 12 Kohlenstoffatomen und vorzugsweise einen linearen oder verzweigten Alkylenrest mit 1 bis 6 Kohlenstoffatomen darstellt.

8. Verfahren gemäß einem der Ansprüche 1 bis 7, **dadurch gekennzeichnet, dass** die mittlere Konzentration des Reaktionsmediums an Dinitril im Rahmen eines industriellen Verfahrens in kontinuierlichem Betrieb zwischen 10 Gew.-% und 45 Gew.-% an Gewicht liegt.

9. Verfahren gemäß einem der Ansprüche 1 bis 8, **dadurch gekennzeichnet, dass** es in Gegenwart von LiOH, NaOH, KOH, RbOH, CsOH und ihren Gemischen durchgeführt wird.

10. Verfahren gemäß einem der Ansprüche 1 bis 9, **dadurch gekennzeichnet, dass** das flüssige Reaktionsmedium wenigstens teilweise wässrig ist und das Wasser eine Menge von 1 Gew.-% bis 25 Gew.-%, bezogen auf das Gewicht des gesamten Reaktionsmediums, darstellt und vorzugsweise zwischen 1 und 15 Gew.-% an Gewicht liegt.

11. Verfahren gemäß einem der Ansprüche 1 bis 10, **dadurch gekennzeichnet, dass** das Reaktionsmedium als Ergänzung oder als Ersatz des Wassers wenigstens ein weiteres Lösungsmittel vom Typ Alkohol umfasst.

12. Verfahren gemäß Anspruch 11, **dadurch gekennzeichnet, dass** das Reaktionsmedium einen Alkohol umfasst, der ausgewählt ist unter Methanol, Ethanol, Propanol, Isopropanol, Butanol und den Gemischen besagter Verbindungen.

13. Verfahren gemäß einem der Ansprüche 1 bis 12, **dadurch gekennzeichnet, dass** das Reaktionsmedium Diamin umfasst, das durch die Hydrierung miterzeugt wird.

14. Verfahren gemäß Anspruch 13, **dadurch gekennzeichnet, dass** in kontinuierlichem Betrieb die mittlere Konzentration des Aminonitrils und/oder des Diamins in dem Reaktionsmedium zwischen 35 Gew.-% und 90 Gew.-% liegt, bezogen auf das Gewicht des gesamten Lösungsmittels, das in besagtem Reaktionsmedium enthalten ist, und stärker bevorzugt zwischen 45 Gew.-% und 89 Gew.-% an Gewicht liegt.

15. Verfahren gemäß einem der Ansprüche 1 bis 14, **dadurch gekennzeichnet, dass** das Reaktionsmedium flüssigen oder gelösten Ammoniak umfasst.

16. Verfahren gemäß einem der Ansprüche 1 bis 15, **dadurch gekennzeichnet, dass** der Ammoniak 0 Gew.-% bis 50 Gew.-% des Reaktionsmediums und vorzugsweise 0 % bis 15 % darstellt

17. Verfahren gemäß einem der Ansprüche 1 bis 10 und 13 bis 16, **dadurch gekennzeichnet, dass** die Menge an Alkali- oder Erdalkalimetallhydroxid größer oder gleich 0,1 mol/kg Katalysator ist, vorzugsweise zwischen 0,1 und 2 mol/kg Katalysator und noch stärker bevorzugt zwischen 0,2 und 1,0 mol/kg Katalysator liegt.

18. Verfahren gemäß einem der Ansprüche 11 und 12, **dadurch gekennzeichnet, dass** die Menge an Alkali- oder Erdalkalimetallhydroxid größer oder gleich 0,05 mol/kg Katalysator ist, vorzugsweise zwischen 0,1 und 10,0 mol/kg Katalysator und stärker bevorzugt zwischen 1,0 und 8,0 mol/kg Katalysator liegt.

19. Verfahren gemäß einem der Ansprüche 1 bis 18, **dadurch gekennzeichnet, dass** es bei einer Temperatur kleiner oder gleich 150 °C, vorzugsweise kleiner oder gleich 120 °C und stärker bevorzugt zwischen der Raumtemperatur (etwa 20 °C) und 100 °C durchgeführt wird.

20. Verfahren gemäß einem der Ansprüche 1 bis 19, **dadurch gekennzeichnet, dass** es unter einem Wasserstoffdruck zwischen 0,10 und 10 MPa durchgeführt wird.

## Claims

1. Process for the partial hydrogenation of a dinitrile into the corresponding aminonitrile, in a liquid medium, **characterized in that** the process is performed in the presence of a Raney nickel or a Raney cobalt catalyst containing at least one metal chosen from copper, silver and gold and in the presence of an alkali metal hydroxide or alkaline-earth metal hydroxide.

2. Process according to Claim 1, **characterized in that** the weight ratio (Cu + Ag + Au)/ Ni or (Cu + Ag + Au)/Co of the catalyst used is between 0.05% and 10% and preferably between 0.1% and 5%.

3. Process according to either of Claims 1 and 2, **characterized in that** the catalyst contains one or more additional dopants chosen from titanium, chromium, iron, zirconium, vanadium, manganese, bismuth, tantalum, rhodium, ruthenium, iridium, platinum, palladium, niobium, hafnium and rare-earth elements, and also cobalt when the catalyst is Raney nickel and nickel when the catalyst is Raney cobalt.

4. Process according to one of Claims 1 to 3, **characterized in that** the amount of dopant other than copper, silver and gold which the catalyst contains represents from 0% to 5% by weight relative to the weight of the nickel or cobalt.

5. Process according to one of Claims 1 to 4, **characterized in that** the catalyst used is preferably a Raney nickel.

6. Process according to one of Claims 1 to 5, **characterized in that** the catalyst is used in the form of grains.

7. Process according to one of Claims 1 to 6, **characterized in that** it applies to the dinitrile substrates of formula (I):
NC―R―CN (I)
in which R represents a linear or branched alkylene group containing from 1 to 12 carbon atoms and preferably a linear or branched alkylene radical containing from 1 to 6 carbon atoms.

8. Process according to one of Claims 1 to 7, **characterized in that** the average concentration of dinitrile in the reaction medium in the context of an industrial process in continuous operation is between 10% and 45% on a weight for weight basis.

9. Process according to one of Claims 1 to 8, **characterized in that** it is carried out in the presence of LiOH, NaOH, KOH, RbOH or CsOH and mixtures thereof.

10. Process according to one of Claims 1 to 9, **characterized in that** the liquid reaction medium is at least partially aqueous and the water represents an amount of from 1% to 25% by weight relative to the weight of the total reaction medium and is preferably between 1% and 15% on a weight for weight basis.

11. Process according to one of Claims 1 to 10, **characterized in that** the reaction medium comprises, in addition to or in replacement for the water, at least one other solvent of alcohol type.

12. Process according to Claim 11, **characterized in that** the reaction medium comprises an alcohol chosen from methanol, ethanol, propanol, isopropanol and butanol and mixtures of the said compounds.

13. Process according to one of Claims 1 to 12, **characterized in that** the reaction medium comprises diamine which is coproduced by the hydrogenation.

14. Process according to Claim 13, **characterized in that** the average concentration of aminonitrile and/or of diamine in the reaction medium in a continuous regime is between 35% and 90% by weight relative to the total weight of the solvent included in said reaction medium and is more preferably between 45% and 89% on a weight for weight basis.

15. Process according to one of Claims 1 to 14, **characterized in that** the reaction medium comprises liquid or dissolved ammonia.

16. Process according to one of Claims 1 to 15, **characterized in that** the ammonia represents from 0% to 50% by weight of the reaction medium and preferably from 0% to 15%.

17. Process according to one of Claims 1 to 10 and 13 to 16, **characterized in that** the amount of alkali metal hydroxide or alkaline-earth metal hydroxide is greater than or equal to 0.1 mol/kg of catalyst, preferably between 0.1 and 2 mol/kg of catalyst and even more preferably between 0.2 and 1.0 mol/kg of catalyst.

18. Process according to either of Claims 11 and 12, **characterized in that** the amount of alkali metal hydroxide or alkaline-earth metal hydroxide is greater than or equal to 0.05 mol/kg of catalyst, preferably between 0.1 and 10.0 mol/kg of catalyst and more preferably between 1.0 and 8.0 mol/kg of catalyst.

19. Process according to one of Claims 1 to 18, **characterized in that** it is carried out at a temperature of less than or equal to 150°C, preferably less than or equal to 120°C and more preferably between room temperature (about 20°C) and 100°C.

20. Process according to one of Claims 1 to 19, **characterized in that** it is carried out at a hydrogen pressure of between 0.10 and 10 MPa.
